# EUROPEAN PATENT SPECIFICATION

(11) **EP 3 927 425 B1**
(45) Date of publication and mention of the grant of the patent: **12.07.2023**
(21) Application number: 19726520.0
(22) Date of filing: 20.02.2019
(51) Int. Cl.: A61K 9/00, A61K 9/51, A61M 37/00, C12N 13/00, C12N 15/87

(54) **ROTARY MAGNETIC ACTUATION SYSTEM**
MAGNETISCHES DREHBETÄTIGUNGSSYSTEM
SYSTÈME D'ACTIONNEMENT MAGNÉTIQUE ROTATIF

(43) Date of publication of application: 29.12.2021
(73) Proprietor: Sabanci Üniversitesi, 34956 Tuzla/Istanbul (TR); Sabanci Üniversitesi Nanoteknoloji Arastirma Ve Uygulama Merkezi Sunum, 34956 Istanbul (TR); KOC Universitesi, 34450 Istanbul (TR)
(72) Inventor: AKGÖNÜL, Sarp, 34956 Tuzla/Istanbul (TR); ZUVIN, Merve, 34956 Tuzla/Istanbul (TR); SEVGEN, Mükerrem, Ilker, 34956 Tuzla/Istanbul (TR); KOSAR, Ali, 34956 Tuzla/Istanbul (TR); GÖZÜACIK, Devrim, 34956 Tuzla/Istanbul (TR); KUTLU, Özlem, 34956 Tuzla/Istanbul (TR); YAGCI ACAR, Havva, 34450 Sariyer/Istanbul (TR)
(74) Representative: Sevinç, Erkan
(86) International application number: PCT/TR2019/050115
(87) International publication number: WO 2020/171783

(56) References cited:
- US-A1- 2006 228 421
- US-A1- 2012 288 505
- Dace Avotina: "INFLUENCE OF THE TIME-PHASE VARIED MAGNETIC FIELD ON THE NUCLEIC ACIDS DELIVERY INTO THE CANCER CELLS", Doctoral Thesis for obtaining the degree of Doctor of Medicine Speciality Theoretical Medicine, 1 January 2016 (2016-01-01), XP055608330, Retrieved from the Internet: URL:https://pdfs.semanticscholar.org/cef1/ c115db34fb44b614556217a5b1c904c12eb7.pdf [retrieved on 2019-07-24]
- DACE VAINAUSKA ET AL: "A novel approach for nucleic acid delivery into cancer cells", MEDICINA (KAUNAS), vol. 48, no. 6, 1 January 2012 (2012-01-01), pages 324-329, XP055608280,
- CH. DAHMANI ET AL: "Rotational magnetic pulses enhance the magnetofection efficiency in vitro in adherent and suspension cells", JOURNAL OF MAGNETISM AND MAGNETIC MATERIALS, vol. 332, 1 April 2013 (2013-04-01), pages 163-171, XP055608287, AMSTERDAM, NL ISSN: 0304-8853, DOI: 10.1016/j.jmmm.2012.12.029

## Description

### Technical Field of the Invention

The present invention relates to a system which is suitable for gene delivery into cells. In particular, the present invention relates to a system utilizing magnetofection for enhancing the efficiency in such gene delivery.

### Background of the Invention

With the advances in nanotechnology, health-related gene therapy applications have gained both variety and importance. Gene therapy involves replacing a gene, which is losing its function, or introducing an exogeneous gene to re-functionalize the damaged gene [1]-[4].

The exogenous gene is to be transported into cells in vectors, which are classified as viral and non-viral vectors. Adenovirus, lentivirus, and adeno-associated viruses are commonly used as viral vectors [5]-[7]. While viral vectors are efficient carriers, they might cause damage to the targeted cell and the surrounding tissue because of their toxicity.

On the other hand, thanks to their reduced toxicity, non-viral vectors emerged as an alternative. In this context, cationic lipids and polymers are commonly used non-viral vectors [8]. They are regarded as suitable carriers for genes since they can be conjugated with different particles and functionalized with coatings [9]-[10].

Magnetic nanoparticles are proven to be good candidates for this task [11]-[13]. The works of Eslaminejad Touba *et al.* and Stephen *et al.* demonstrate the iron oxide nanoparticles as advantageous among magnetic nanoparticles for drug/gene delivery in therapeutics and diagnostics of various diseases because of their biocompatibility, functionality and physical properties [14]-[15]. By being in superparamagnetic regime, iron oxide nanoparticles are effective when an external magnetic field is applied. Upon removal of the magnetic field, the iron oxide nanoparticles can be removed from an environment/body because they will have no remnant magnetization [16].

Transferring genes via nanoparticles using magnetic fields is called "magnetofection" [17]-[19]. For being used in this technique, nanoparticles are substantially coated for DNA binding and are sent to the cell. Then, the nanoparticles get interacted with cell membrane, taken up by endocytosis.

The expression takes place after internalization [17], [20]. Polyethyleneimine (PEI) is a conventional and well established transfection agent, as a cationic polymer. However, it is known to be harmful to cells [21]-[22]. Coating nanoparticles with a polymer such as polyethyleneimine and using them with magnetic fields increase the transfection efficiency, while reducing the toxicity effects [23]-[24].

Many different magnetic systems have been used for gene transfer. The popular ones are oscillating magnet arrays, placing magnets under the culture plates and magnet arrays [23], [25], [26]. McBain et al. [25] used an oscillating magnet array and reported that human lung epithelial cells were effectively transfected. They reported positive effects of the system on viability.

Lu et al. [26] used a staggered magnet array and placed two magnets underneath the culture plates. Two adjacent magnets interfered with each other and had a negative effect on transfection. In Lu et al [26], because the two magnets were affected by each other, they claimed that the efficiency was more in between. Accordingly, the cells, which were located above the magnets, experienced a uniform magnetic field, while the cells in the other wells of the plate were exposed to non-uniform magnetic fields. The group concluded that the non-uniform magnetic field was more suitable for in vivo studies.

In our previous study, we showed that polyethyleneimine (PEI) coated superparamagnetic (here: iron oxide) nanoparticles (PEI-SPIONs) can efficiently transfer green fluorescent protein tagged DNA (GFP-DNA) under varying magnetic fields. In that study, the transfection agents were kept in contact with the cells with an 8-hour incubation, while the device was in operation. The viability was improved, but the transfection yield was still lower than the PEI.

The Chinese patent application CN 1029 11865 A relates to magnetofection using a couple of plates provided with magnetic strips, said plates being rotated around a vertical axis. In order to create a "uniform" magnetic field around cells to be subjected to transfection, one of said plates is placed below a vessel which includes said cells, and the other one of the plates is placed above said vessel. The document does not include any information on cell viability, nor on the extent of transfection efficiency available with such system.

US 2006/228421 A1 discloses a device for moving magnetically responsive nanoparticles through a membrane adapted to rotate at least one magnet in a manner to cause repetitive variation in the magnetic field gradient produced at the membrane by magnets. However, the document is silent about an alignment of magnets, in which a pole of each of said magnets faces an opposite pole of a subsequent magnet.

US 2012/288505 A1 provides a method of delivery of a therapeutic agent to a target cell; the method comprises targeting particles comprising the therapeutic agent to the cell using magnetic means to apply a magnetic force to the particles so as to tend to move the particles towards or away from the magnetic means and at the same time moving the magnetic means. However, it does not concern aligning multiple magnets based on their magnetic poles, nor mentions the term pole or any linguistic equivalent thereof.

Dace Avotina [27] discloses a magnetofection device wherein a time-phase varying magnetic field is based on a permanent magnets plate orbital rotation in a plane parallel to the cell culture plate. It is disclosed that magnetofection efficiency depending on the magnets rotation frequency - the most important characterising parameter of the time-phase varied magnetic field. The disclosed system includes a magnet carrier rotating around an axis and in said system the magnets have their respective orbits which are off the axis. Dace Vainauska et al. [28] describes the effects of incubation time under a dynamic magnetic field and a rotation frequency of magnets on transfection efficiency. Rotating property of an orbital shaker was used for creating a dynamic magnetic field. However, [27] and [28] are both silent about an alignment of magnets, in which a pole of each of said magnets faces an opposite pole of a subsequent magnet; and furthermore, there is no teaching in [27] and [28] that magnets are arranged in a substantially circular configuration such that gravitational and/or magnetic centers of the magnets substantially form a circle around the axis substantially parallel to the gravity vector when the system is in use. Dahmani et. al. [29] discloses a magnetofection system comprising rotating permanent magnets to generate defined varying magnetic fields. The disclosed magnets of [29] are arranged to be rotated around the wells, instead of below the wells.

### List of reference documents:

[1] C. M. Curtin, E. G. Tierney, K. Mcsorley, S. A. Cryan, G. P. Duffy, and F. J. O'Brien, "Combinatorial gene therapy accelerates bone regeneration: Non-viral dual delivery of VEGF and BMP2 in a collagen-nanohydroxyapatite scaffold", Adv. Healthc. Mater., vol. 4, no. 2, pp. 223-227, 2015.
[2] K. L. Jackson, R. D. Dayton, E. A. Orchard, S. Ju, D. Ringe, G. A. Petsko, L. E. Maquat, and R. L. Klein, "Preservation of forelimb function by UPF1 gene therapy in a rat model of TDP-43-induced motor paralysis", Gene Ther., vol. 22, no. 1, pp. 20-28, 2015.
[3] A. Mangraviti, S. Y. Tzeng, K. L. Kozielski, Y. Wang, Y. Jin, D. Gullotti, M. Pedone, N. Buaron, A. Liu, D. R. Wilson, S. K. Hansen, F. J. Rodriguez, G. D. Gao, F. Dimeco, H. Brem, A. Olivi, B. Tyler, and J. J. Green, "Polymeric nanoparticles for nonviral gene therapy extend brain tumor survival in vivo", ACS Nano, vol. 9, no. 2, pp. 1236-1249, 2015.
[4] A. Georgiadis, Y. Duran, J. Ribeiro, L. Abelleira-Hervas, S. J. Robbie, B. Sünkel-Laing, S. Fourali, A. Gonzalez-Cordero, E. Cristante, M. Michaelides, J. W. B. Bainbridge, A. J. Smith, and R. R. Ali, "Development of an optimized AAV2/5 gene therapy vector for Leber congenital amaurosis owing to defects in RPE65", Gene Ther., vol. 23, no. 12, pp. 857-862, 2016.
[5] S. Palfi, J. M. Gurruchaga, G. Scott Ralph, H. Lepetit, S. Lavisse, P. C. Buttery, C. Watts, J. Miskin, M. Kelleher, S. Deeley, H. Iwamuro, J. P. Lefaucheur, C. Thiriez, G. Fenelon, C. Lucas, P. Brugières, I. Gabriel, K. Abhay, X. Drouot, N. Tani, A. Kas, B. Ghaleh, P. Le Corvoisier, P. Dolphin, D. P. Breen, S. Mason, N. V. Guzman, N. D. Mazarakis, P. A. Radcliffe, R. Harrop, S. M. Kingsman, O. Rascol, S. Naylor, R. A. Barker, P. Hantraye, P. Remy, P. Cesaro, and K. A. Mitrophanous, "Long-term safety and tolerability of ProSavin, a lentiviral vector-based gene therapy for Parkinson's disease: A dose escalation, open-label, phase 1/2 trial", Lancet, vol. 383, no. 9923, pp. 1138-1146, 2014.
[6] R. G. Crystal, "Adenovirus: The First Effective In Vivo Gene Delivery Vector", Hum. Gene Ther., vol. 25, no. 1, pp. 3-11, 2014.
[7] R. E. MacLaren, M. Groppe, A. R. Barnard, C. L. Cottriall, T. Tolmachova, L. Seymour, K. Reed Clark, M. J. During, F. P. M. Cremers, G. C. M. Black, A. J. Lotery, S. M. Downes, A. R. Webster, and M. C. Seabra, "Retinal gene therapy in patients with choroideremia: Initial fi ndings from a phase 1/2 clinical trial", Lancet, vol. 383, no. 9923, pp. 1129-1137, 2014.
[8] P. Agrawal, N. P. Ingle, W. S. Boyle, E. Ward, J. Tolar, K. D. Dorfman, and T. M. Reineke, "Fast, Efficient, and Gentle Transfection of Human Adherent Cells in Suspension", ACS Appl. Mater. Interfaces, vol. 8, no. 14, pp. 8870-8874, 2016.
[9] H. Yin, R. L. Kanasty, A. A. Eltoukhy, A. J. Vegas, J. R. Dorkin, and D. G. Anderson, "Non-viral vectors for gene-based therapy", Nat. Rev. Genet., vol. 15, no. 8, pp. 541-555, 2014.
[10] M. Ramamoorth and A. Narvekar, "Non viral vectors in gene therapy - An overview", J. Clin. Diagnostic Res., vol. 9, no. 1, pp. GE01-GE06, 2015.
[11] X. J. Loh, T.-C. Lee, Q. Dou, and G. R. Deen, "Utilising inorganic nanocarriers for gene delivery", Biomater. Sci., vol. 4, no. 1, pp. 70-86, 2016.
[12] A. A. M. Elsherbini, M. Saber, M. Aggag, A. El-Shahawy, and H. A. A. Shokier, "Magnetic nanoparticle-induced hyperthermia treatment under magnetic resonance imaging", Magn. Reson. Imaging, vol. 29, no. 2, pp. 272-280, 2011.
[13] S. Majidi, F. Zeinali Sehrig, M. Samiei, M. Milani, E. Abbasi, K. Dadashzadeh, and A. Akbarzadeh, "Magnetic nanoparticles: Applications in gene delivery and gene therapy", Artif. Cells, Nanomedicine, Biotechnol., vol. 1401, no. November, pp. 1-8, 2015.
[14] A. M. Eslaminejad Touba, Noureddin Nematollahi-Mahani Seyed, "Glioblastoma Targeted Gene Therapy Based on pEGFP/p53-Loaded Superparamagnetic Iron Oxide Nanoparticles", Curr. Gene Ther., vol. 17, no. 11, pp. 59-69, 2017.
[15] Z. R. Stephen, C. J. Dayringer, J. J. Lim, R. A. Revia, M. V. Halbert, M. Jeon, A. Bakthavatsalam, R. G. Ellenbogen, and M. Zhang, "Approach to Rapid Synthesis and Functionalization of Iron Oxide Nanoparticles for High Gene Transfection", ACS Appl. Mater. Interfaces, vol. 8, no. 10, pp. 6320-6328, 2016.
[16] W. Wu, Z. Wu, T. Yu, C. Jiang, and W. S. Kim, "Recent progress on magnetic iron oxide nanoparticles: Synthesis, surface functional strategies and biomedical applications", Sci. Technol. Adv. Mater., vol. 16, no. 2, pp. 59-69, 2015.
[17] Wahajuddin and S. Arora, "Superparamagnetic iron oxide nanoparticles: Magnetic nanoplatforms as drug carriers", Int. J. Nanomedicine, vol. 7, pp. 3445-3471, 2012.
[18] F. Scherer, M. Anton, U. Schillinger, J. Henke, C. Bergemann, A. Krüger, B. Gänsbacher, and C. Plank, "Magnetofection: Enhancing and targeting gene delivery by magnetic force in vitro and in vivo", Gene Ther., vol. 9, no. 2, pp. 102-109, 2002.
[19] C. Plank, F. Scherer, U. Schillinger, C. Bergemann, and M. Anton, "Magnetofection: enhancing and targeting gene delivery with superparamagnetic nanoparticles and magnetic fields", J. Liposome Res., vol. 13, no. 1, pp. 29-32, 2003.
[20] L. Zhang, Y. Li, J. C. Yu, Y. Y. Chen, and K. M. Chan, "Assembly of polyethylenimine-functionalized iron oxide nanoparticles as agents for DNA transfection with magnetofection technique", J. Mater. Chem. B, vol. 2, no. 45, pp. 7936-7944, 2014.
[21] H. Lv, S. Zhang, B. Wang, S. Cui, and J. Yan, "Toxicity of cationic lipids and cationic polymers in gene delivery", J. Control. Release, vol. 114, no. 1, pp. 100-109, 2006.
[22] S. M. Moghimi, P. Symonds, J. C. Murray, A. C. Hunter, G. Debska, and A. Szewczyk, "A two-stage poly(ethylenimine)-mediated cytotoxicity: Implications for gene transfer/therapy", Mol. Ther., vol. 11, no. 6, pp. 990-995, 2005.
[23] L. Prosen, S. Hudoklin, M. Cemazar, M. Stimac, U. Lampreht Tratar, M. Ota, J. Scancar, R. Romih, and G. Sersa, "Magnetic field contributes to the cellular uptake for effective therapy with magnetofection using plasmid DNA encoding against Mcam in B16F10 melanoma in vivo", Nanomedicine (Lond), vol. 11, no. 6, pp. 627-641, 2016.
[24] O. Oral, T. Cιkιm, M. Zuvin, O. Unal, H. Yagci-Acar, D. Gozuacik, and A. Ko 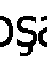 ar, "Effect of Varying Magnetic Fields on Targeted Gene Delivery of Nucleic Acid-Based Molecules", Ann. Biomed. Eng., vol. 43, no. 11, pp. 2816-2826, 2015.
[25] S. C. McBain, U. Griesenbach, S. Xenariou, A. Keramane, C. D. Batich, E. W. F. W. Alton, and J. Dobson, "Magnetic nanoparticles as gene delivery agents: Enhanced transfection in the presence of oscillating magnet arrays," Nanotechnology, vol. 19, no. 40, pp. 59-69, 2008.
[26] Y. C. Lu, F. Y. Chang, S. J. Tu, J. P. Chen, and Y. H. Ma, "Cellular uptake of magnetite nanoparticles enhanced by NdFeB magnets in staggered arrangement," J. Magn. Magn. Mater., vol. 427, no. June 2016, pp. 71-80, 2017.
[27] Dace Avotina, "INFLUENCE OF THE TIME-PHASE VARIED MAGNETIC FIELD ON THE NUCLEIC ACIDS DELIVERY INTO THE CANCER CELLS", Doctoral Thesis for obtaining the degree of Doctor of Medicine Speciality Theoretical Medicine, (20160101)
[28] DACE VAINAUSKA ET AL, "A novel approach for nucleic acid delivery into cancer cells", MEDICINA (KAUNAS), (20120101), vol. 48, no. 6, pages 324 - 329
[29] CH. DAHMANI ET AL, "Rotational magnetic pulses enhance the magnetofection efficiency in vitro in adherent and suspension cells", JOURNAL OF MAGNETISM AND MAGNETIC MATERIALS, AMSTERDAM, NL, (20130401), vol. 332, ISSN 0304-8853, pages 163 - 171

### Objects of the Invention

Primary object of the present invention is to overcome the abovementioned shortcomings of the prior art.

Another object of the present invention is to provide a transfection system with enhanced efficiency in gene delivery.

Another object of the present invention is to provide a transfection system with high cell viability.

Another object of the present invention is to provide a system enabling an expedited transfection.

A further object of the present invention is to provide a simple, compact and low cost transfection system.

### Summary of the Invention

The present invention proposes a compact system for magnetic particle coupled gene delivery into the targeted cells. The gene delivery is achieved with the introduction of a rotational system comprising one or more magnets, thereby providing an external energy input onto the magnetic particles thus providing further mobilization thereto.

The present concept reduces the average delivery (i.e. transfection) time of the desired material (here: genes) while eliminating the necessity of commonly applied incubation procedure for gene delivery. As the rotation of the magnet integrated system introduces an interchanging magnetic field direction with varying magnitudes, the desired mobilization of the gene coupled magnetic particles can be achieved. The rotation of the magnet integrated component, which exerts an interchanging magnetic field direction, offers mobilization of the gene coupled magnetic particles in all directions. The mobilization realized in all directions increases the probability of target cell-magnetic particles interaction at the cell periphery compared to the permanent magnetic field systems, where the magnetic particles interact with the target cells along a singular direction.

### Brief Description of the Figures

The figures, whose brief explanation is herewith provided, are solely intended for providing a better understanding of the present invention and are as such not intended to define the scope of protection or the context in which said scope is to be interpreted in the absence of the description.
Fig.1 shows a simplified plan view of a magnet carrier on which a magnet is attached for being employed in a system, emphasizing that the magnet is disposed to remain within a first projection of a surface on which magnetofection is to be applied.
Fig.2 shows a simplified plan view of another exemplary magnet carrier on which a plurality of magnets is attached for being employed in a system according to the present invention, emphasizing that the plurality of magnets is disposed to remain within the first projection.
Fig.3 shows a simplified plan view of an exemplary magnet carrier on which a plurality of magnets is attached, exemplifying a distance between the locus of gravitational and/or magnetic centers of one or more magnets with respect to a width or radius of the first projection.
Fig.4 is a perspective view of an exemplary vessel to be used with the system according to the present invention, emphasizing its surface to be provided with cells to be subjected to magnetofection. The surface can be circular, e.g. in the form of a closed disc, as in a Petri dish.
Fig.5 is a simplified perspective view of a system according to the present invention, exemplifying the relative positioning and distance between a surface on which cells (to be subjected to magnetofection) are placed, and the gravitational/magnetic centers of magnet(s) to be moved/turned below the surface.
Fig.6 is a section view of the exemplary system shown in the Fig.4, along a rotation axis around which the magnet(s) are to be moved/turned; emphasizing the absence of any further magnetic field to be exerted from above the surface, thus the magnetic field from "below" the surface can be fully utilized.

### Detailed Description of the Invention

The present invention proposes a system (100) provided with a plurality of magnets (2), which can be provided on a magnet carrier (3).

The system (100) is suitable for subjecting cells (e.g. a group of living cells) placed onto a surface (s1) (which can be a substantially planar surface) of a vessel (4) when such vessel (4) is introduced into the system (100) and then the system (100) is run (i.e. the system is brought into use), to magnetofection using a fluid including magnetizable particles to be brought into contact (i.e. interaction) with such cells. In other words, the system (100) is suitable for subjecting cells placed onto a surface (s1) (e.g. onto a substantially planar surface) of a vessel (4), to magnetofection with a fluid including magnetizable particles to be brought into interaction with such cells.

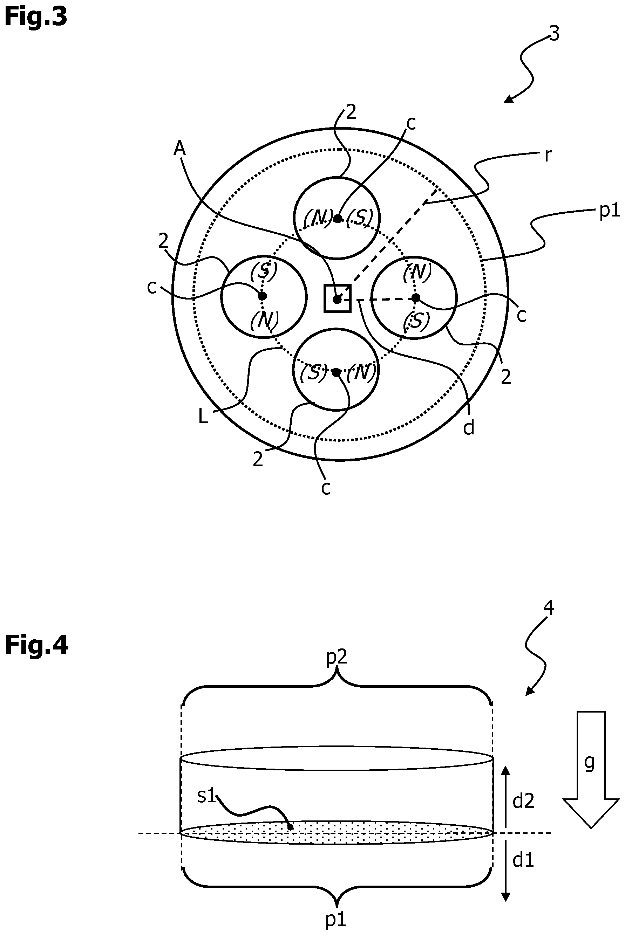

Such vessel (4), such fluid, such cells, and such magnetizable particles can be obtained independently from such vessel (4), e.g. from commercial suppliers; and then introduced to the system (100). Thus, the vessel, the fluid, the cells and magnetizable particles are not constitute main features of the system (100) according to the present invention. Perspective view of a vessel (4) suitable for being used with the system (100) according to the present invention is depicted in Fig.4.

The system (100) includes a surface carrier (1) to support such vessel (4), such that the surface (s1) has a first projection (p1) in a first direction (d1) which is substantially parallel to a direction of the gravity vector (g), and a second projection (p2) in a second direction (d2) which is substantially opposite to said first direction (d1).

The system (100) further includes a plurality of magnets (2) (as mentioned above) to provide a magnetic field at a vicinity of the surface carrier (1) for, in use, magnetizing such particles; said magnets (2) being arranged to, in use, be moved substantially parallel to said surface (s1).

Said magnets (2) are disposed within the first projection (p1), and the system (100) is adapted over a layout of said magnets (2) to provide that, when such vessel (4) is introduced to the system (100) and the system (100) is run:
a) either the system (100) does not include any further magnet in said second projection (p2), or
b) a first magnetic field strength available by said magnets (2) from the first projection (p1) to such surface (s1), is greater than any further magnetic field strength available from the second projection (p2) to such surface (s1).

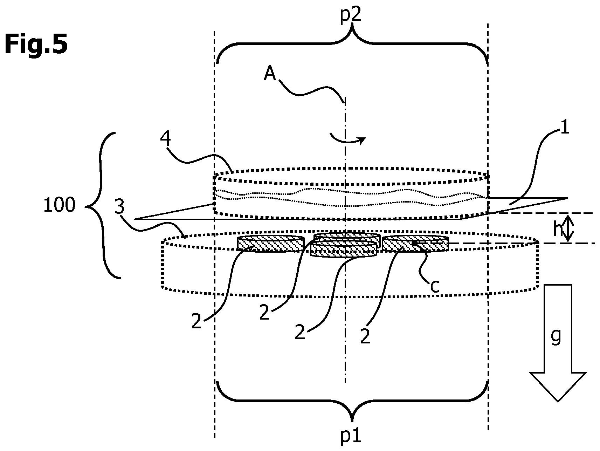

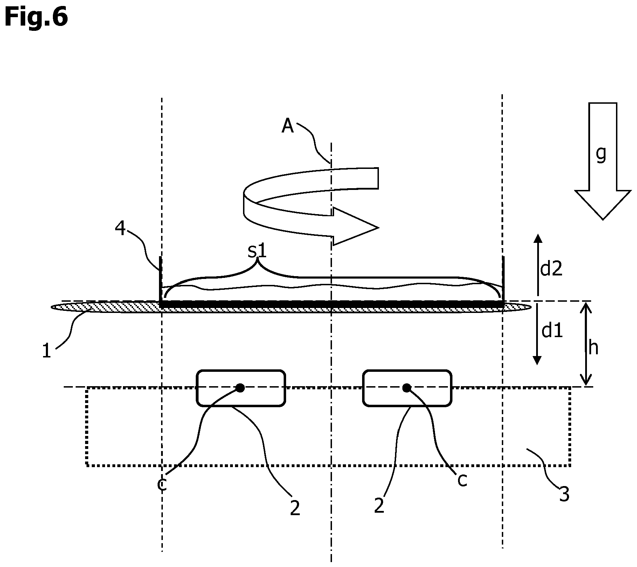

A perspective view and a side view of a system (100) according to the present invention, visually exemplifying resulting respective layouts between the surface carrier (1) (thus surface (s1)) and the magnet(s) (2) are depicted in Fig.5 and Fig.6, respectively.

By the above mentioned set of features, when in use, the system (100) substantially prevents the particles (i.e. magnetizable particles) from leaving a base (i.e. surface (s1)) of a vessel (4) introduced into the system (100). This is because the (first) magnetic field strength caused by the magnets (2) disposed within the first projection (p1), is not challenged by any (further) magnetic field strength applying from the second projection (p2) to such surface; since either such further magnetic field strength is absent, or the first magnetic field strength is greater than any such "further" magnetic strength. Thereby the system (10) can exert a "net" magnetic force in the first direction (d1) (i.e. a downward force, in the direction of gravity (g)) onto magnetic particles, and thus the magnetic particles can be driven closer to cells and used with enhanced efficiency.

Accordingly, when the system (100) is in use, the cells on the surface (s1) are swept with said particles by being magnetically pulled in the first direction (d1) (i.e. downwards), and simultaneously by being driven substantially orthogonal to said first direction (d1) (i.e. being forced to move on the first surface (s1)). As a result, both of the alternatives (a) and (b) enable that, when the system (100) is in use, particles sweep the surface (s1) instead of being substantially distributed throughout a depth of the fluid; thereby enhancing the efficiency of magnetofection into the cells provided on the surface (s1). Thus, the system (100) enables that a minimized amount of particles suffices for achieving an effective magnetofection. The system further enables the achievement of an effective magnetofection in a minimized time of contacting the particles to the cells.

The present invention thus further proposes a method for magnetofection. The method includes the following features:
- provision of a vessel (4) having a surface (s1) (which can be a substantially planar surface) onto which cells are placed, provision of a fluid including magnetizable particles in contact (i.e. interaction) with said cells;
- supporting said vessel (4) with a surface carrier (1), such that the surface (s1) has a first projection (p1) in a first direction (d1) which is substantially parallel to a direction of the gravity vector (g), and a second projection (p2) in a second direction (d2) which is substantially opposite to said first direction (d1);
- provision of a plurality of magnets (2) to provide a magnetic field at a vicinity of the surface carrier (1) for magnetizing said particles; moving said magnets (2) substantially parallel to said surface (s1), thereby driving said particles on said surface (s1);
- arranging that said magnets (2) are disposed within the first projection (p1) and adapting the system over a layout of said magnets (2) to provide that,
   i. either it is avoided that the second projection (p2) does not include any further magnets, or
   ii. a first magnetic field strength applied by said magnets (2) from the first projection (p1) to such surface (s1), is kept greater than any further magnetic field strength available from the second projection (p2) to such surface (s1).

In other words, a first magnetic field strength exerted from the first projection (p1) to such surface (s1) should be greater than second magnetic field strength exerted from a second projection (p2) of such surface (s1) in a second direction (d2) which is substantially opposite to said first direction (d1) (i.e. even in a case where any further magnet is disposed "above" the surface (s1)). Considering that a magnetic field to subject the cells to magnetofection with an enhanced efficiency by driving the particles on the surface (s1) is available by magnetically "pulling" the particles from "below" (from the first projection (p1) of the surface (s1)), a "net" magnetic force exerted onto the particles has to be parallel to the direction of the gravity vector (g). Although the high performance magnetofection can be achieved with both of the alternatives (i) and (ii), the first alternative (i) can be considered even more favorable in order to achieve this goal with a minimized cost related to obtaining the magnets (2).

In the system (100) according to the present invention, said magnets (2) are arranged to be turned around an axis (A) substantially parallel to a gravity vector (g) to drive such particles. Accordingly, a continuous rotational movement of the magnets (2) is achieved, which provides a simplified system (100) and a facilitated mode of operation thereto, along with prolonged service life. Especially in the case where a vessel (4) with a round shaped surface (s1, base) is used with the system, the particles can be continuously rotated around a geometrical center of the surface (s1) without sudden direction changes due to colliding side walls of the vessel.

In order to achieve such arrangement in a more preferable way, the system (100) can include a magnet carrier (3) for supporting said magnets (2). Said magnet carrier (3) can be arranged to be rotated around said axis (A), such that the magnets (2) are turned around said axis (A). Inclusion of such magnet carrier (3) even better simplifies the system (100), especially in the case where a plurality of magnets (2) is employed, by enabling the movement of the magnets (2) over a movement of a shared magnet carrier (3).

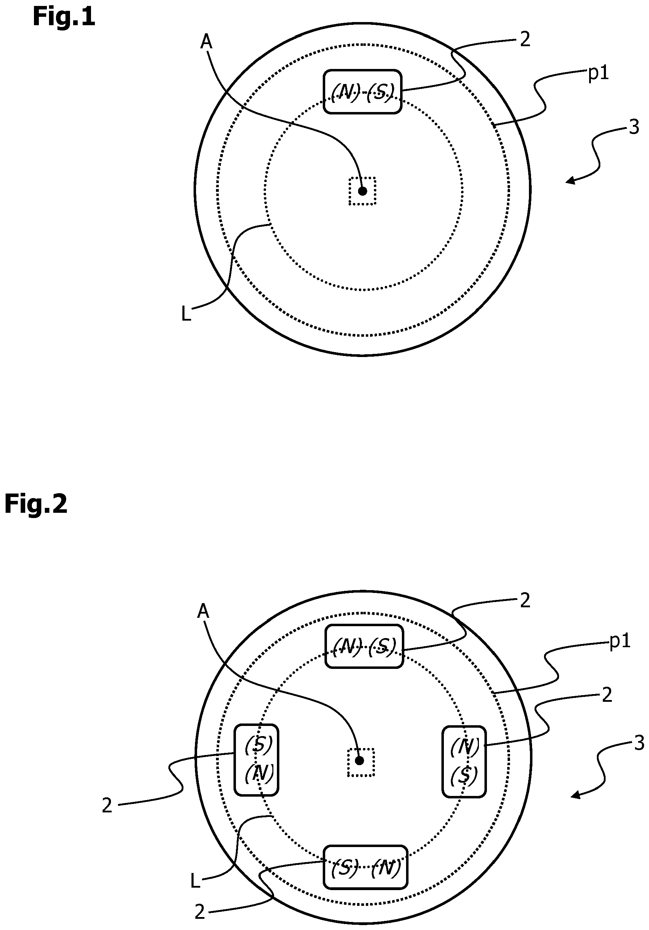

Plan views of exemplary magnet carriers (3) provided with a plurality of magnets (2) are depicted in Fig.2 and Fig.3.

Accordingly, in the method according to the present invention, said moving of said magnets (2) can be performed by turning said magnets (2) around an axis (A) substantially parallel to a gravity vector (g); preferably by rotating a magnet carrier (3) supporting said magnets (2), around said axis (A) such that the magnets (2) are turned around said axis (A).

The system (100) is provided with a plurality of said magnets (2) arranged in an alignment, such that a pole of each of said magnets (2) faces an opposite pole of a subsequent magnet (2) on said alignment. In particular when the movement of said plurality of magnets (2) is performed in a direction substantially following a sequence of such opposite poles, orientations of particles can be randomized whilst sweeping the surface (s1) with said particles; so the particles can approach the cells from varying aspects and an even enhanced efficiency, rate and uniformity of magnetofection can be achieved throughout the surface (s1). The "uniformity" of magnetofection is not to be confused with a uniformity of magnetic field. The movable magnets (2) here instead provide a non-uniform (randomized) and non-stagnant magnetic field throughout the surface (s1), which in use serves for an increased exposure of the cells to the particles. This is because that, in use, the cells are not exposed to particles from a single direction, instead the orientations of mobilized particles change as being subjected to varying/randomized magnetic fields and poles as the alternating magnetic poles of the plurality of magnets (2) pass "below" them.

Furthermore, said alignment of the magnets is arranged in a substantially circular configuration, such that gravitational and/or magnetic centers (c) of said magnets (2) substantially form a circle around an axis (A) substantially parallel to a gravity vector (g). Thereby, the above mentioned randomization can be performed in a continuous manner, which results in a high complexity in mobilized particle orientations sweeping the surface (s1), and thereby achieving an expedited magnetofection with even higher efficiency. In use, this further enables exertion of varying magnetic poles and varying extent of magnetic field strength onto particles in a regulated manner by turning the magnets (2) around said axis (A) at a predetermined rotation frequency, thus easily foresee the movement of the magnetic field throughout the surface (s1).

Accordingly, the method according to the present invention includes arrangement of a plurality of said magnets (2) in an alignment such that a pole of each of said magnets (2) faces an opposite pole of a subsequent magnet (2) on said alignment; said alignment is arranged in a substantially circular configuration, such that gravitational and/or magnetic centers (c) of said magnets (2) substantially form a circle around said axis (A).

Said substantially circular configuration of alignment can preferably be arranged, such that a locus (L) of the gravitational and/or magnetic centers (c) of said magnets (2) is disposed at (or within) an annular region on the first projection (p1). Said annular region can have an inner radius and an outer radius around said axis (A). Said inner radius can correspond to one third of a radius (r) of said first projection (p1), and said outer radius can correspond to two third of said radius (r) of said first projection (p1). Thus, a distance (d) between the locus (L) and the axis (A) can remain between said inner radius and outer radius both of which being smaller than the radius (r) of the first projection (p1). With this embodiment, magnetofection can be effectively achieved even when a low number of magnets (2) are circularly aligned, magnetic field strength of which being (substantially uniformly) available to majority of particles throughout a width or diameter of the surface (s1) of a vessel (4). Plan view of an exemplary magnet carrier (3) which fulfills the requirements of this embodiment is depicted in the Fig.3.

Accordingly, the method according to the present invention can include arrangement of said alignment in a substantially circular configuration, such that gravitational and/or magnetic centers (c) of said magnets (2) substantially form a circle around said axis (A); and a locus (L) of the gravitational and/or magnetic centers (c) of said magnets (2) is disposed at an annular region on the first projection (p1); said annular region having, around said axis (A), an inner radius corresponding to one third of a radius (r) of said first projection (p1), and an outer radius corresponding to two third of said radius (r) of said first projection (p1).

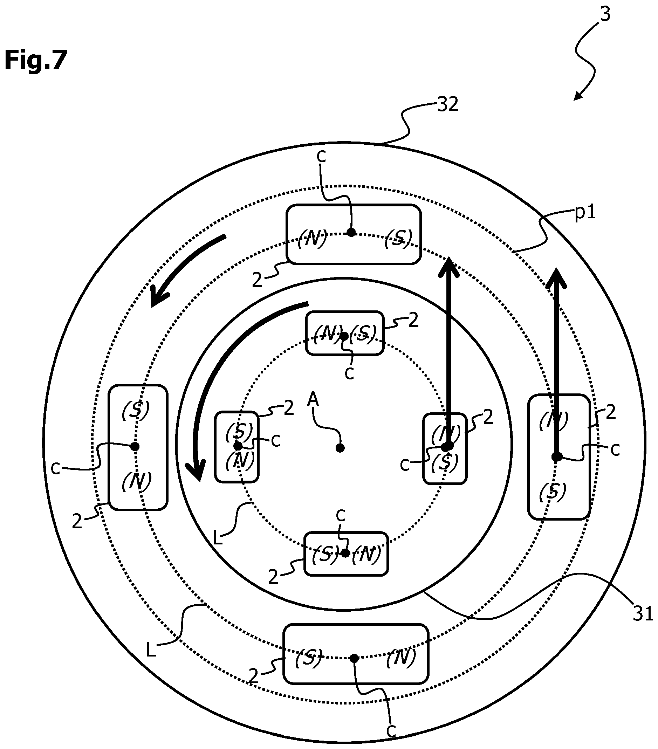

In an embodiment according to the present invention, the system (100) can include a plurality (set) of coaxially arranged magnet carriers (3) to be rotated around the axis (A) at different speeds (i.e. different numbers of revolutions per minute), such that comparable or substantially equal linear speeds of gravitational and/or magnetic centers (c) of magnets can be achieved on different magnet carriers (3). Thereby, in use, particles at different distances to the axis (A) can be subjected to more comparable conditions, and the predictability of the system (100) is even better enhanced. To this end, one or more magnets (2) are to be positioned on an inner magnet carrier (31) (i.e. having a narrower locus (L)), and further one or more magnets (2) are positioned on an outer magnet carrier (32) (i.e. having a wider locus (L) in comparison with that at an inner magnet carrier (31)). Such inner magnet carrier (31) is to be rotated faster (with faster revolutions) when compared to an outer magnet carrier (32) to achieve comparable or equal linear speeds at said narrower and wider loci (L). Plan view of an exemplary set with a plurality (here: two) coaxial set of magnet carriers (3) is schematized in the Fig.7; relatively high and low revolution speeds and comparable or equal linear velocities at different magnet carriers (31 and 32) are represented with curved bold arrows of different lengths, and straight bold arrows of comparable (here: equal) lengths, respectively. As visualized in the Fig.7 over different corpulence at magnets (2) on different magnet carriers (31 and 32 here), magnets (2) of different magnetic strengths can be employed and preferably magnets (2) can be distributed to different magnet carriers constituting common magnet carrier (3), in accordance with their magnetic strengths.

The system according to the present invention can be arranged to, in use, provide a linear velocity of up to 1500 cm/min at a gravitational and/or magnetic center (c) of said magnets (2). Considering that a translational velocity of a magnetizable particle within a magnetic field of a magnet (2) can be determined by a linear velocity of the magnet (2), and considering that cell membranes can be damaged when being impacted by a particle with a high velocity, such values of linear velocity can be considered acceptable for magnetofection without causing mechanical damage on cell membranes.

The system can preferably be arranged to, in use, provide a linear velocity within the range between 500 cm/min and 1000 cm/min at the gravitational and/or magnetic center (c) of said magnets (2). Considering that very low translational velocity at a particle under magnetic field of a magnet (2) can cause that a desired extent of magnetofection is achieved only after long exposure times; the preferred lower limit of 500 cm/min results in completion of magnetofection in reasonably short times which are always much shorter when compared to those available with prior art technologies.

Accordingly, in the method according to the present invention, said moving of the magnets (2) can correspond to a linear speed of up to 1500 cm/min at a gravitational and/or magnetic center (c) of said magnets (2); said linear speed being preferably kept within the range between 500 cm/min and 1000 cm/min.

The system according to any one of the claims 1 to 5, wherein said magnets (2) are arranged such that a magnetic field of up to 1.5 T is exerted to the vicinity of the surface carrier (1). Higher magnetic field strengths are considered unnecessary for the function of the system (100); thus this embodiment of the system (100) allows reduced production costs without compromising a magnetofection performance available with the system (100). Preferably, said magnetic field is within the range between 50 mT and 1 T. 50 mT is sufficient to provide a substantial mobility to particles, and complete mobility of particles at linear velocities of the gravitational and/or magnetic centers (c) of the magnets (2) is considered available with 1 T.

Accordingly, the method according to the present invention can include the selection of said magnets (2) such that a magnetic field of up to 1.5 T is exerted to the vicinity of the surface carrier (1); preferably said magnetic field is kept within the range between 50 mT and 1 T.

Such system can be arranged to, when such vessel (4) is introduced into the system (100), provide a vertical distance (h) between such surface (s1) and a gravitational and/or magnetic center (c) of a magnet (2) within the range between 3 cm and 4 cm. As already deducible with the results of the exemplary experimental results, a more favorable magnetofection performance in shortest times is availed using a distance within this range, by employing magnet(s) (2) providing a magnetic field strength at the surface (s1) corresponding to the extents mentioned above.

Accordingly, in the method according to the present invention, vertical distance (h) between such surface (s1) and a gravitational and/or magnetic center (c) of a magnet (2) is kept within the range between 3 cm and 4 cm.

The method according to the present invention preferably includes employing superparamagnetic iron oxide nanoparticles coated with an anionic or cationic polymer, such as SPIONs with a cationic surface, in particular SPIONs with PEI coating (e.g. PEI-SPIONs) as magnetizable particles, because of their favorable properties already mentioned within the present specification, enhancing their suitability for being used in magnetofection. Said nanoparticles can be loaded with a therapeutic cargo, such that said nanoparticles carry a therapeutic cargo (e.g. gene and/or drug). The magnetizable particles are preferred to provide an acceptable level of stability when in colloidal systems, and measures for arrangement of the magnetizable particles to be colloidally stable is evident to a person skilled in colloidal chemistry or colloidal systems. For more clearly showing the advantages of the system of method according to the present invention, the magnetizable particles selected for being employed in the exemplary experiments, are PEI-SPIONs.

### EXEMPLARY EXPERIMENTAL WORK FOR PROOF OF CONCEPT

The employment of magnetizable particles (here: PEI coated SPIONs) under magnetic fields in nucleic acid delivery has been investigated to overcome the disadvantages of conventional methods of transfection by gene therapy. In this regard, with the proposed system, better tissue localization and higher transfection efficiency can be achieved, and this system can be easily tested in both vitro and in vivo studies.

The magnetizable particles used in the exemplary experimental study had a median hydrodynamic diameter of 84 nm. It is clear to a person skilled in the art of magnetofection, it is sufficient to select magnetizable particles of having a size allowing to penetrate biological pores on cell membranes, generally accepted as e.g. corresponding to median hydrodynamic diameters within ranges of 8 nm to 50 nm, and 20 nm to 200 nm, for in vivo studies and in vitro studies, respectively.

An experimental setup is prepared which represents a possible embodiment of the system (100) according to the present invention. A petri dish is commercially obtained to be used as a vessel (4) with a disk-shaped planar surface (s1) having a diameter of 10 cm.

Neodymium magnets each providing a maximum magnetic field strength of 300 mT are secured to a single, disk-shaped magnet carrier (3) which is to be rotated around a vertical axis (A) when in use. Magnetic field strength is measured throughout a vertical projection on the magnet carrier (3). Said measurement is performed at a single distance from the magnet carrier (3), corresponding to that of the surface (s1) (here: roughly to that of the surface carrier (1)), and values ranging between 60 mT and 230 mT were recorded.

### The exemplary actuation system:

The experimental apparatus is provided with a movable (rotary) mechanism integrated with the magnet carrier. The rotary mechanism is rotated using a motor (e.g. a DC motor, here: 12V). In order to try and find an optimal distance with the magnets used in the system, adjustable stages were used for placing the vessel(s) (here: petri dishes) to be subjected to magnetic field of the magnets.

The stages were made of plexiglass, yet any other material with mechanical stability sufficient to weigh the vessels can be used.

A plurality of magnets are used in the experimental setup, in order to utilize interaction of consecutive magnets. The magnets were secured onto a table in a circular configuration. The configuration is arranged such that each pole of each magnet face the opposite pole of an adjacent magnet; so, consecutive magnets in a row constituted a -(N-S-N-S)- configuration.

Stationary components and the carrier of the system were chosen to be fabricated with manufacturing methods such as the laser cutting and 3D printing techniques, yet the fabrication method of the stationary components does clearly not affect the functioning of the experimental system.

Magnetic field magnitudes of the magnets and the variations in magnetic field depending on the distance between the table and the sample were measured/followed using a commercially available gaussmeter (Hirst Magnetic Instruments Ltd.).

### Proof of Concept experiments:

A varying magnetic field throughout a vertical projection over the magnets at the level of the surface carrier (which roughly corresponds to a level of the surface of the vessel) is generated by moving the magnets (here: by rotating the magnet carrier which a rotary table).

Taking the behavior of the magnetic field strength measurement device into consideration, a non-uniform magnetic field magnitude ranging from 2 to 60mT could be measured throughout the magnet carrier. To visualize the effect, magnetizable particles (those of visually observable sizes, here: iron dusts) were introduced into the vessel, and the mobilization of the particles was observed with varying distances between the vessel and the magnet integrated component.

When the vessel containing iron dusts is placed on the surface carrier over the magnet carrier, without moving the magnets (without rotating the magnet carrier), it is observed that the iron dusts are influenced by each magnet's own field and aggregate around vertical projections of gravitational or magnetic center of each individual magnet as expected, and also clustered near the edges. As the vessel was incrementally moved in a direction opposite to the gravity vector (upwards, away from the surface carrier), the effect of the magnets was observed to be coherent due to the evenly distribution of dusts on the plate.

Then, the effect of the moving of magnets (here: turning) on the iron dusts was investigated by rotating the magnet carrier (here: counter clock-wise when observed downwards). First, the behavior of iron dusts above the rotating magnets at 2.5 cm distance was examined, and the results demonstrate a recurring mobilization pattern of the particles coherent with the magnets rotational direction. It can be observed that at 2.5 cm distance iron dusts arrange successively then mobilize as the magnet rotates, followed by a rotation towards the magnets' new position, and finally aforementioned recurring mobilization. Since the effect of magnetic field was observed to be strong at this distance, various distances ranging from 2 to 3.5 cm were examined for the cell experiments. The objective of moving the magnets relative to the surface (e.g. via rotating the magnet carrier) is to allow as many nanoparticles as possible to enter into the cells, which are attached to the surface; thus an optimized distance was investigated for higher transfection rates. The optimal distance is clearly dependent to the magnetic field strength exerted onto the surface by the magnet(s).

In order to investigate the uniformity, it was shown that a uniform transfection can be obtained under non-uniform magnetic field (in the sense of time-dependent variability in magnetic field strength due to changing positions of magnets, at each certain zone on the surface) by finding a magnet strength dependent optimal distance between the magnets and culture plate:
- Cell experiments at various distances were performed for this task.
- At a smallest distance of 2 cm for the exemplary experimental setup, a more concentrated cell population was seen around the magnet, and
- a cell population was found distributed throughout the entire 10 cm diameter surface of the vessel (Petri plate) at a distance (h) of 3.5 cm from the plate center (axis (A)). It is clear that this optimal distance (h) is valid for the characteristics of magnets employed in the present experimental setup, and higher optimal distance values are to be expected for a system (100) with a stronger magnetic field at gravitational and/or magnetic center(s) (c) of magnet(s) (2).

The cell experiments were carried out with 3 different magnetizable particle samples, namely:
- polyethyleneimine (abbreviated as PEI),
- polyethyleneimine coated superparamagnetic iron oxide nanoparticles without the introduction of magnetic field (abbreviated as PS wo), and
- polyethyleneimine coated superparamagnetic iron oxide nanoparticles under moving (rotary) magnetic field (abbreviated as PS rot).

Initially, the effect of post transfection time was investigated. After 1 h magnetofection and 12 h transfection time, the viability of cells was highly deteriorated. Considering viability experiments, the control sample was also added among PEI, PS wo and PS rot. To optimize the viability, 6 - 3 - 1 h post transfection times and speeds of 120 and 60 rpm were investigated. Considering that the magnets had a substantially circular layout on the magnet carrier, with a distance of ca. 5 cm between the rotation axis and gravitational and/or magnetic center of (each) magnet; said speeds correspond to linear velocities at gravitational and/or magnetic center of (each) magnet of ca. 1884 cm/min and 942 cm/min, respectively.

Also, the effect of distance (h) between the magnet carrier (roughly corresponding to the gravitational and/or magnetic center of each magnet) and the surface of the vessel (roughly corresponding to the surface carrier) was investigated to obtain a uniform distribution checked with dust. For this, 2 - 2.5 - 3 - 3.5 cm distances were explored. After the experiments, the parameters were set to 3 h of transfection time and 60 rpm in order to obtain decreased cell death and efficient transfection. Then, 1 h of transfection time was investigated, and further enhancement of cell viability and efficient transfection could be achieved. After the experiments, 300 cells are counted from each plate, and the transfection efficiencies are examined. According to results of 1 h of post transfection, efficiency of PEI is remained low (around 2%), while that of PS rot at a distance (h) of 3.5 cm is 45%.

Interventions, which could be introduced to the transfection protocol, were investigated using MCF7 cells, which are known to be resistant to transfection. Even with MCF7 cells, the (post-) transfection time of 8 hours available in the prior art technologies, was reduced to 1 hour. It is concluded that the necessity of an incubator during the experiment was eliminated thanks to the system and method according to the present invention. The viability also significantly increased when the transfection agents were washed out from the cells at the end of 1 hour of actuation. When the transfection efficiencies were examined, it was proven that effective transfection could be performed even within a short time (1 hour) with the system (100). Accordingly, GFP-DNA transfer to MCF7 cell line with high efficiency was achieved with magnetofection. As a result, it was proven that the proposed system and method are extremely effective by significantly reducing the gene delivery time and eliminating the necessity of the common incubation procedure.

Several advantages of the proposed system compared to the available ones can be listed as follows:
- An important advantage of this invention arises from the ability of system to achieve the desired transfection within shorter post transfection times (reduced to even 1 hour). This improvement also eliminates any (exposure time dependent) toxic effects of nanoparticles.
- Known methods include putting the cell plate and the device into an incubator together. However, with the system according to the present invention, a highly effective hyperthermia transfection and viability can be achieved without the use of an incubator due to the smaller transfection time which eliminates the incubation procedure for sustaining the viability. This reduces the complexity and steps/equipment requirements to achieve transfection, clearly and dramatically reducing the related labor and equipment costs.
- Since transfection agents are effective for gene delivery, toxicity of these agents are important drawbacks. Due to the high number of target cell-magnetic particles interaction achieved by this system, smaller number of agents can be introduced into the medium to be contacted with cells, and for a shorter exposure time; thus a safe gene delivery with cost efficiency can be achieved.

The results showed that the use of the presented concept benefitting from magnet induced varying magnetic field, decreased the cell death by reducing the transfection time while increasing the transfection efficiency.

The employment of PEI coated SPIONs (PEI-SPIONs) under magnetic fields in nucleic acid delivery has been investigated to overcome the disadvantages of conventional methods of transfection by gene therapy. In this regard, with the use of the presented system, better tissue localization and higher transfection efficiency can be achieved in shorter times. Furthermore, the invention can easily be used in academic research for both in vitro and in vivo biological studies.

Using this apparatus and method, we investigated magnetofection under non-uniform magnetic fields with the aim of high cell viability and high transfection rate. Based on the results on MCF-7 cell line and PEI-SPIONs, we achieved short transfection time and high transfection rates without having cell death. In the light of the known success of PEI in causing exogenous DNA to enter the cells resulting transfection, we determined the PEI group as control. Accordingly, different with or without magnetic field experiments were conducted on PEI-SPIONs with cells, and their efficiency was assessed. GFP-DNA transfer to MCF7 cells was obtained. Longer transfection time such as 8 h resulted in higher rate of cell death due to longer exposure to agents. High rotational speeds resulted in decreased cell viability. The results of the application of variable magnetic field onto the PEI-SPIONs showed resemblance to that of the cells with PEI agents solely without the magnetic field. After 48 hours of incubation period (for expression to occur), the viability and transfection tests were performed. At a distance (h) of 3.5 cm, it was observed that magnets produce a more efficient magnetic field on the cell culture plate, which can be deduced from gene expression patterns similar to the iron dust patterns. Uniform distribution was obtained and in vitro gene transfer was observed.

The references to any methods of treatment disclosed above are to be interpreted as references to the compounds, pharmaceutical compositions and medicaments of the present invention for use in a method for treatment of the human or animal body by therapy or for diagnosis.

Thus the following objects are achieved by the present invention:
- overcoming the abovementioned shortcomings of the prior art, and
- provision of a transfection system and method with enhanced efficiency in expedited gene delivery, high cell viability, which is simple and low cost.

### List of reference numerals:

- 1: surface carrier
- 2: magnet(s)
- 3: magnet carrier
- 4: vessel
- A: axis
- c: gravitational and/or magnetic center of a magnet
- d1: first direction
- d2: second direction
- g: gravity vector
- h: vertical distance between a surface and a gravitational and/or magnetic center of a magnet (substantially corresponding to a relative "height" of the surface)
- L: locus of gravitational and/or magnetic centers of magnets
- p1: first projection of the surface
- p2: second projection of the surface
- s1: surface

## Claims

1. A system (100)
for subjecting cells placed onto a surface (s1) of a vessel (4), to magnetofection with a fluid including magnetizable particles to be brought into interaction with such cells;
said system (100) including:
- a surface carrier (1) to support such vessel (4), such that when the system (100) is in use, the surface (s1) has a first projection (p1) in a first direction (d1) which is substantially parallel to a direction of the gravity vector (g), and a second projection (p2) in a second direction (d2) which is substantially opposite to said first direction (d1); and
- a plurality of magnets (2) to provide a magnetic field at a vicinity of the surface carrier (1) for, in use, magnetizing the particles; said magnets (2) being arranged to, in use, be moved substantially parallel to said surface (s1) and drive the particles by being turned around an axis (A) substantially parallel to the gravity vector (g),
said magnets (2) are disposed within the first projection (p1), and the system (100) is adapted over a layout of said magnets (2) to provide that, when such vessel (4) is introduced to the system (100) and the system (100) is run:
- either the system (100) does not include any further magnet in said second projection (p2), or
- a first magnetic field strength available by said magnets (2) from the first projection (p1) to such surface (s1), is greater than any further magnetic field strength available from the second projection (p2) to such surface (s1);
wherein
the magnets (2) are arranged in an alignment, such that a pole of each of said magnets (2) faces an opposite pole of a subsequent magnet (2) on said alignment; and said alignment is arranged in a substantially circular configuration, such that gravitational and/or magnetic centers (c) of said magnets (2) substantially form a circle around the axis (A) substantially parallel to a gravity vector (g) when the system (100) is in use; and the movement of said plurality of magnets (2) is to be performed in a direction substantially following a sequence of such opposite poles.

2. The system (100) according to the claim 1, wherein the system (100) includes a magnet carrier (3) for supporting said magnets (2) for said driving of particles, and said magnet carrier (3) is arranged to be rotated around said axis (A), such that the magnets (2) are turned around said axis (A).

3. The system according to any of the claims 1 or 2, wherein said alignment is in a substantially circular configuration, such that gravitational and/or magnetic centers (c) of said magnets (2) substantially form a circle around said axis (A); and a locus (L) of the gravitational and/or magnetic centers (c) of said magnets (2) is disposed at an annular region on the first projection (p1); said annular region having, around said axis (A), an inner radius corresponding to one third of a radius (r) of said first projection (p1), and an outer radius corresponding to two third of said radius (r) of said first projection (p1).

4. The system according to any one of the claims 1 to 3, arranged to, when in use, provide a linear velocity of up to 1500 cm/min at a gravitational and/or magnetic center (c) of said magnets (2), said linear velocity preferably being within the range between 500 cm/min and 1000 cm/min.

5. The system according to any one of the claims 1 to 4, wherein said magnets (2) are arranged such that a magnetic field of up to 1.5 T is exerted to the vicinity of the surface carrier (1); preferably said magnetic field is within the range between 50 mT and 1 T.

6. The system according to the claim 5, arranged to, when such vessel (4) is introduced into the system (100), provide a vertical distance between such surface (s1) and a gravitational and/or magnetic center (c) of a magnet (2) within the range between 3 cm and 4 cm.

7. A method for magnetofection, including
- provision of a vessel (4) having a surface (s1) onto which a group of cells are placed, provision of a fluid including magnetizable particles in interaction with said cells;
- supporting said vessel (4) with a surface carrier (1), such that the surface (s1) has a first projection (p1) in a first direction (d1) which is substantially parallel to a direction of the gravity vector (g), and a second projection (p2) in a second direction (d2) which is substantially opposite to said first direction (d 1);
- provision of a plurality of magnets (2) to provide a magnetic field at a vicinity of the surface carrier (1) for magnetizing said particles; moving said magnets (2) substantially parallel to said surface (s1), thereby driving said particles on said surface (s1);
- arranging that said magnets (2) are disposed within the first projection (p1) and adapting the system over a layout of said magnets (2) to provide that,
- either it is avoided that the second projection (p2) does not include any further magnets, or
- a first magnetic field strength applied by said magnets (2) from the first projection (p1) to such surface (s1), is kept greater than any further magnetic field strength available from the second projection (p2) to such surface (s1);
wherein
the magnets (2) are turned around an axis (A) substantially parallel to a gravity vector (g) to move said magnets (2); and
the method further includes arrangement of the magnets (2) in an alignment such that a pole of each of said magnets (2) faces an opposite pole of a subsequent magnet (2) on said alignment;
said alignment being arranged in a substantially circular configuration, such that gravitational and/or magnetic centers (c) of said magnets (2) substantially form a circle around an axis (A) substantially parallel to a gravity vector (g); and the movement of said plurality of magnets (2) is performed in a direction substantially following a sequence of such opposite poles.

8. The method according to the claim 7, wherein said turning of the magnets (2) around the axis (A) is performed by rotating a magnet carrier (3) supporting said magnets (2), around said axis (A).

9. The method according to any of the claims 7 or 8, including arrangement of said alignment in a substantially circular configuration, such that gravitational and/or magnetic centers (c) of said magnets (2) substantially form a circle around said axis (A); and a locus (L) of the gravitational and/or magnetic centers (c)of said magnets (2) is disposed at an annular region on the first projection (p1); said annular region having, around said axis (A), an inner radius corresponding to one third of a radius (r) of said first projection (p1), and an outer radius corresponding to two third of said radius (r) of said first projection (p1).

10. The method according to any one of the claims 7 to 9, wherein said moving of the magnets (2) corresponds to a linear speed of up to 1500 cm/min at a gravitational and/or magnetic center (c) of said magnets (2); said linear speed being preferably kept within the range between 500 cm/min and 1000 cm/min.

11. The method according to any one of the claims 7 to 10, including the selection of said magnets (2) such that a magnetic field of up to 1.5 T is exerted to the vicinity of the surface carrier (1); preferably said magnetic field is kept within the range between 50 mT and 1 T.

12. The method according to any one of the claims 7 to 11, wherein a vertical distance between such surface (s1) and a gravitational and/or magnetic center (c) of a magnet (2) is kept within the range between 3 cm and 4 cm.

13. The method according to any one of the claims 7 to 12, wherein the method further includes the employment of, superparamagnetic iron oxide nanoparticles coated with an anionic or cationic polymer, as said magnetizable particles.

## Patentansprüche

1. System (100),
um Zellen, die auf einer Oberfläche (s1) eines Gefäßes (4) platziert sind, einer Magnetofektion mit einem Fluid zu unterziehen, das magnetisierbare Partikel umfasst, die in Wechselwirkung mit solchen Zellen gebracht werden sollen; wobei das System (100) Folgendes umfasst:
- einen Oberflächenträger (1) zum Tragen dieses Gefäßes (4), sodass, wenn das System (100) in Gebrauch ist, die Oberfläche (s1) Folgendes aufweist: einen ersten Vorsprung (p1) in einer ersten Richtung (d1), die im Wesentlichen parallel zu einer Richtung des Schwerkraftvektors (g) ist, und einen zweiten Vorsprung (p2) in einer zweiten Richtung (d2), die im Wesentlichen entgegengesetzt zu der ersten Richtung (d1) ist; und
- eine Vielzahl von Magneten (2), um ein Magnetfeld in einer Umgebung des Oberflächenträgers (1) bereitzustellen, um in Gebrauch die Partikel zu magnetisieren; wobei die Magneten (2) derart angeordnet sind, dass sie in Gebrauch im Wesentlichen parallel zu der Oberfläche (s1) bewegt werden und die Partikel antreiben, indem sie um eine Achse (A) gedreht werden, die im Wesentlichen parallel zu dem Schwerkraftvektor (g) ist,
wobei die Magneten (2) in dem ersten Vorsprung (p1) angeordnet sind und das System (100) über einer Anordnung der Magneten (2) eingestellt wird, um dafür zu sorgen, dass, wenn dieses Gefäß (4) in das System (100) eingeführt wird und das System (100) betrieben wird:
- entweder das System (100) keinen weiteren Magneten in dem zweiten Vorsprung (p2) enthält, oder
- eine erste Magnetfeldstärke, die durch die Magneten (2) von dem ersten Vorsprung (p1) zu dieser Oberfläche (s1) verfügbar ist, größer als jede weitere Magnetfeldstärke ist, die von dem zweiten Vorsprung (p2) zu dieser Oberfläche (s1) verfügbar ist;
wobei
die Magneten (2) in einer derartigen Anordnung angeordnet sind, dass ein Pol von jedem der Magneten (2) einem entgegengesetzten Pol eines nachfolgenden Magneten (2) in der Anordnung zugewandt ist; und wobei die Anordnung in einer im Wesentlichen kreisförmigen Konfiguration angeordnet ist, sodass die Gravitations- und/oder Magnetzentren (c) der Magneten (2) im Wesentlichen einen Kreis um die Achse (A) bilden, die im Wesentlichen parallel zu einem Schwerkraftvektor (g) ist, wenn das System (100) in Gebrauch ist; und wobei die Bewegung der Vielzahl von Magneten (2) in einer Richtung erfolgen soll, die im Wesentlichen einer Abfolge dieser entgegengesetzten Pole folgt.

2. System (100) nach Anspruch 1, wobei das System (100) einen Magnetträger (3) zum Tragen der Magneten (2) zum Antreiben der Partikel umfasst und wobei der Magnetträger (3) um die Achse (A) drehbar angeordnet ist, sodass die Magneten (2) um die Achse (A) gedreht werden.

3. System nach einem der Ansprüche 1 oder 2, wobei sich die Anordnung in einer im Wesentlichen kreisförmigen Konfiguration befindet, sodass die Gravitations- und/oder Magnetzentren (c) der Magneten (2) im Wesentlichen einen Kreis um die Achse (A) bilden; und wobei ein Lokus (L) der Gravitations- und/oder Magnetzentren (c) der Magneten (2) in einem ringförmigen Bereich auf dem ersten Vorsprung (p1) angeordnet ist; wobei der ringförmige Bereich um die Achse (A) einen inneren Radius, der einem Drittel eines Radius (r) des ersten Vorsprungs (p1) entspricht, und einen äußeren Radius, der zwei Dritteln des Radius (r) des ersten Vorsprungs (p1) entspricht, aufweist.

4. System nach einem der Ansprüche 1 bis 3, das so beschaffen ist, dass es in Gebrauch an einem Gravitations- und/oder Magnetzentrum (c) der Magneten (2) eine lineare Geschwindigkeit von bis zu 1500 cm/min bereitstellt, wobei die lineare Geschwindigkeit vorzugsweise im Bereich zwischen 500 cm/min und 1000 cm/min liegt.

5. System nach einem der Ansprüche 1 bis 4, wobei die Magneten (2) so beschaffen sind, dass ein Magnetfeld von bis zu 1,5 T auf die Umgebung des Oberflächenträgers (1) ausgeübt wird; wobei das Magnetfeld vorzugsweise im Bereich zwischen 50 mT und 1 T liegt.

6. System nach Anspruch 5, das so beschaffen ist, dass, wenn dieses Gefäß (4) in das System (100) eingesetzt ist, ein vertikaler Abstand zwischen dieser Oberfläche (s1) und einem Gravitations- und/oder Magnetzentrum (c) eines Magneten (2) im Bereich zwischen 3 cm und 4 cm vorgesehen ist.

7. Verfahren zur Magnetofektion, umfassend:
- Bereitstellen eines Gefäßes (4), das eine Oberfläche (s1) aufweist, auf der eine Gruppe von Zellen platziert wird, Bereitstellen eines Fluids, das magnetisierbare Partikel in Wechselwirkung mit den Zellen umfasst;
- Stützen des Gefäßes (4) mit einem Oberflächenträger (1), sodass die Oberfläche (s1) Folgendes aufweist: einen ersten Vorsprung (p1) in einer ersten Richtung (d1), die im Wesentlichen parallel zu einer Richtung des Schwerkraftvektors (g) ist, und einen zweiten Vorsprung (p2) in einer zweiten Richtung (d2), die im Wesentlichen entgegengesetzt zu der ersten Richtung (d1) ist;
- Bereitstellen einer Vielzahl von Magneten (2), um ein Magnetfeld in einer Umgebung des Oberflächenträgers (1) zur Magnetisierung der Partikel bereitzustellen; Bewegen der Magneten (2) im Wesentlichen parallel zu der Oberfläche (s1), wodurch die Partikel auf der Oberfläche (s1) angetrieben werden;
- Sicherstellen, dass die Magneten (2) in dem ersten Vorsprung (p1) angeordnet sind, und Einstellen des Systems über einer Anordnung der Magneten (2), um dafür zu sorgen, dass
- entweder vermieden wird, dass der zweite Vorsprung (p2) keine weiteren Magneten umfasst, oder
- eine erste Magnetfeldstärke, die durch die Magneten (2) von dem ersten Vorsprung (p1) auf diese Oberfläche (s1) ausgeübt wird, größer als jede weitere Magnetfeldstärke gehalten wird, die von dem zweiten Vorsprung (p2) zu dieser Oberfläche (s1) verfügbar ist;
wobei
die Magneten (2) um eine Achse (A) gedreht werden, die im Wesentlichen parallel zu einem Schwerkraftvektor (g) ist, um die Magneten (2) zu bewegen; und
das Verfahren ferner das Anordnen der Magneten (2) in einer derartigen Anordnung umfasst, dass ein Pol von jedem der Magneten (2) einem entgegengesetzten Pol eines nachfolgenden Magneten (2) in der Anordnung zugewandt ist; wobei die Anordnung in einer im Wesentlichen kreisförmigen Konfiguration angeordnet ist, sodass die Gravitations- und/oder Magnetzentren (c) der Magneten (2) im Wesentlichen einen Kreis um eine Achse (A) bilden, die im Wesentlichen parallel zu einem Schwerkraftvektor (g) ist;
und wobei die Bewegung der Vielzahl von Magneten (2) in einer Richtung erfolgt, die im Wesentlichen einer Abfolge dieser entgegengesetzten Pole folgt.

8. Verfahren nach Anspruch 7, wobei das Drehen der Magneten (2) um die Achse (A) erfolgt, indem ein Magnetträger (3), der die Magneten (2) trägt, um die Achse (A) gedreht wird.

9. Verfahren nach einem der Ansprüche 7 oder 8, umfassend das Anordnen der Anordnung in einer im Wesentlichen kreisförmigen Konfiguration, sodass die Gravitations- und/oder Magnetzentren (c) der Magneten (2) im Wesentlichen einen Kreis um die Achse (A) bilden; und wobei ein Lokus (L) der Gravitations- und/oder Magnetzentren (c) der Magneten (2) in einem ringförmigen Bereich auf dem ersten Vorsprung (p1) angeordnet ist; wobei der ringförmige Bereich um die Achse (A) einen inneren Radius, der einem Drittel eines Radius (r) des ersten Vorsprungs (p1) entspricht, und einen äußeren Radius, der zwei Dritteln des Radius (r) des ersten Vorsprungs (p1) entspricht, aufweist.

10. Verfahren nach einem der Ansprüche 7 bis 9, wobei die Bewegung der Magneten (2) einer linearen Geschwindigkeit von bis zu 1500 cm/min an einem Gravitations- und/oder Magnetzentrum (c) der Magneten (2) entspricht; wobei die lineare Geschwindigkeit vorzugsweise im Bereich zwischen 500 cm/min und 1000 cm/min gehalten wird.

11. Verfahren nach einem der Ansprüche 7 bis 10, umfassend das derartige Auswählen der Magneten (2), dass ein Magnetfeld von bis zu 1,5 T auf die Umgebung des Oberflächenträgers (1) ausgeübt wird; wobei das Magnetfeld vorzugsweise im Bereich zwischen 50 mT und 1 T gehalten wird.

12. Verfahren nach einem der Ansprüche 7 bis 11, wobei ein vertikaler Abstand zwischen dieser Oberfläche (s1) und einem Gravitations- und/oder Magnetzentrum (c) eines Magneten (2) im Bereich zwischen 3 cm und 4 cm gehalten wird.

13. Verfahren nach einem der Ansprüche 7 bis 12, wobei das Verfahren ferner die Verwendung von superparamagnetischen Eisenoxid-Nanopartikeln, die mit einem anionischen oder kationischen Polymer beschichtet sind, als magnetisierbare Partikel umfasst.

## Revendications

1. - Système (100)
pour soumettre des cellules placées sur une surface (s1) d'un récipient (4) à une magnétofection avec un fluide comprenant des particules magnétisables devant être amenées en interaction avec de telles cellules ;
ledit système (100) comprenant :
- un support de surface (1) pour supporter un tel récipient (4), de telle sorte que, lorsque le système (100) est en cours d'utilisation, la surface (s1) présente une première projection (p1) dans une première direction (d1) qui est sensiblement parallèle à une direction du vecteur de gravité (g), et une seconde projection (p2) dans une seconde direction (d2) qui est sensiblement opposée à ladite première direction (d1) ; et
- une pluralité d'aimants (2) pour fournir un champ magnétique à un voisinage du support de surface (1) pour, lors de l'utilisation, magnétiser les particules, lesdits aimants (2) étant agencés pour, lors de l'utilisation, être déplacés sensiblement parallèlement à ladite surface (s1) et entraîner les particules en tournant autour d'un axe (A) sensiblement parallèle au vecteur de gravité (g),
lesdits aimants (2) sont disposés à l'intérieur de la première projection (p1), et le système (100) est adapté sur une disposition desdits aimants (2) pour assurer que, lorsqu'un tel récipient (4) est introduit dans le système (100) et que le système (100) fonctionne :
- soit le système (100) ne comprend pas un quelconque autre aimant dans ladite seconde projection (p2), soit
- une première intensité de champ magnétique disponible par lesdits aimants (2) de la première projection (p1) à une telle surface (s1) est supérieure à toute autre intensité de champ magnétique disponible de la seconde projection (p2) à une telle surface (s1) ;
dans lequel les aimants (2) sont agencés dans un alignement, de telle sorte qu'un pôle de chacun desdits aimants (2) fait face à un pôle opposé d'un aimant suivant (2) sur ledit alignement ; et ledit alignement est agencé dans une configuration sensiblement circulaire, de telle sorte que des centres de gravité et/ou magnétiques (c) desdits aimants (2) forment sensiblement un cercle autour de l'axe (A) sensiblement parallèle à un vecteur de gravité (g) lorsque le système (100) est en cours d'utilisation ; et le mouvement de ladite pluralité d'aimants (2) doit être effectué dans une direction suivant sensiblement une séquence de tels pôles opposés.

2. - Système (100) selon la revendication 1, dans lequel le système (100) comprend un support d'aimants (3) pour supporter lesdits aimants (2) pour ledit entraînement de particules, et ledit support d'aimants (3) est agencé pour tourner autour dudit axe (A), de telle sorte que les aimants (2) tournent autour dudit axe (A).

3. - Système selon l'une quelconque des revendications 1 ou 2, dans lequel ledit alignement est dans une configuration sensiblement circulaire, de telle sorte que les centres de gravité et/ou magnétiques (c) desdits aimants (2) forment sensiblement un cercle autour dudit axe (A) ; et un lieu (L) des centres de gravité et/ou magnétiques (c) desdits aimants (2) est disposé à une région annulaire sur la première projection (p1), ladite région annulaire ayant, autour dudit axe (A), un rayon interne correspondant à un tiers d'un rayon (r) de ladite première projection (p1), et un rayon externe correspondant aux deux-tiers dudit rayon (r) de ladite première projection (p1).

4. - Système selon l'une quelconque des revendications 1 à 3, agencé pour, lors de l'utilisation, fournir une vitesse linéaire allant jusqu'à 1500 cm/min à un centre de gravité et/ou magnétique (c) desdits aimants (2), ladite vitesse linéaire étant, de préférence, à l'intérieur de la plage entre 500 cm/min et 1000 cm/min.

5. - Système selon l'une quelconque des revendications 1 à 4, dans lequel lesdits aimants (2) sont agencés de telle sorte qu'un champ magnétique de jusqu'à 1,5 T est exercé au voisinage du support de surface (1) ; de préférence ledit champ magnétique est à l'intérieur de la plage entre 50 mT et 1 T.

6. - Système selon la revendication 5, agencé pour, lorsqu'un tel récipient (4) est introduit dans le système (100), fournir une distance verticale entre une telle surface (s1) et un centre de gravité et/ou magnétique (c) d'un aimant (2) à l'intérieur de la plage entre 3 cm et 4 cm.

7. - Procédé de magnétofection, comprenant :
- la fourniture d'un récipient (4) ayant une surface (s1) sur laquelle un groupe de cellules est placé, la fourniture d'un fluide comprenant des particules magnétisables en interaction avec lesdites cellules ;
- le support dudit récipient (4) avec un support de surface (1), de telle sorte que la surface (s1) a une première projection (p1) dans une première direction (d1) qui est sensiblement parallèle à une direction du vecteur de gravité (g), et une seconde projection (p2) dans une seconde direction (d2) qui est sensiblement opposée à ladite première direction (d1);
- la fourniture d'une pluralité d'aimants (2) pour fournir un champ magnétique à un voisinage du support de surface (1) pour magnétiser lesdites particules ; le déplacement desdits aimants (2) sensiblement parallèlement à ladite surface (s1), entraînant ainsi lesdites particules sur ladite surface (s1);
- l'agencement suivant lequel lesdits aimants (2) sont disposés à l'intérieur de la première projection (p1) et l'adaptation du système sur une disposition desdits aimants (2) pour assurer que,
- soit l'on évite que la seconde projection (p2) ne comporte pas de quelconques autres aimants, soit
- une première intensité de champ magnétique appliquée par lesdits aimants (2) de la première projection (p1) à une telle surface (s1) est maintenue supérieure à toute autre intensité de champ magnétique disponible de la seconde projection (p2) à un telle surface (s1) ;
dans lequel les aimants (2) tournent autour d'un axe (A) sensiblement parallèle à un vecteur de gravité (g) pour déplacer lesdits aimants (2) ; et
le procédé comprend en outre un agencement des aimants (2) dans un alignement de telle sorte qu'un pôle de chacun desdits aimants (2) fait face à un pôle opposé d'un aimant suivant (2) sur ledit alignement ;
ledit alignement étant agencé dans une configuration sensiblement circulaire, de telle sorte que des centres de gravité et/ou magnétiques (c) desdits aimants (2) forment sensiblement un cercle autour d'un axe (A) sensiblement parallèle à un vecteur de gravité (g) ; et le mouvement de ladite pluralité d'aimants (2) est effectué dans une direction suivant sensiblement une séquence de tels pôles opposés.

8. - Procédé selon la revendication 7, dans lequel ladite rotation des aimants (2) autour de l'axe (A) est effectuée par la rotation autour dudit axe (A) d'un support d'aimants (3) supportant lesdits aimants (2).

9. - Procédé selon l'une quelconque des revendications 7 ou 8, comprenant l'agencement dudit alignement dans une configuration sensiblement circulaire, de telle sorte que des centres de gravité et/ou magnétiques (c) desdits aimants (2) forment sensiblement un cercle autour dudit axe (A) ; et un lieu (L) des centres de gravité et/ou magnétiques (c) desdits aimants (2) est disposé à une région annulaire sur la première projection (p1), ladite région annulaire ayant, autour dudit axe (A), un rayon interne correspondant à un tiers d'un rayon (r) de ladite première projection (p1), et un rayon externe correspondant aux deux-tiers dudit rayon (r) de ladite première projection (p1).

10. - Procédé selon l'une quelconque des revendications 7 à 9, dans lequel ledit déplacement des aimants (2) correspond à une vitesse linéaire de jusqu'à 1500 cm/min à un centre de gravité et/ou magnétique (c) desdits aimants (2), ladite vitesse linéaire étant, de préférence, maintenue à l'intérieur de la plage entre 500 cm/min et 1000 cm/min.

11. - Procédé selon l'une quelconque des revendications 7 à 10, comprenant la sélection desdits aimants (2) de telle sorte qu'un champ magnétique de jusqu'à 1,5 T est exercé au voisinage du support de surface (1) ; de préférence ledit champ magnétique est maintenu à l'intérieur de la plage entre 50 mT et 1 T.

12. - Procédé selon l'une quelconque des revendications 7 à 11, dans lequel une distance verticale entre une telle surface (s1) et un centre de gravité et/ou magnétique (c) d'un aimant (2) est maintenue à l'intérieur de la plage entre 3 cm et 4 cm.

13. - Procédé selon l'une quelconque des revendications 7 à 12, dans lequel le procédé comprend en outre l'emploi de nanoparticules d'oxyde de fer superparamagnétique revêtues d'un polymère anionique ou cationique, en tant que lesdites particules magnétisables.
